# EUROPEAN PATENT SPECIFICATION

(11) **EP 1 409 636 B1**
(45) Date of publication and mention of the grant of the patent: **19.04.2006**
(21) Application number: 02703427.1
(22) Date of filing: 05.03.2002
(51) Int. Cl.: C12M 1/28

(54) **BIOLOGICAL SPECIMEN COLLECTION APPARATUS**
SAMMELEINRICHTUNG FÜR BIOLOGISCHEN PROBEN
APPAREIL POUR RECUEILLIR DES ECHANTILLONS BIOLOGIQUES

(30) Priority: 05.03.2001 CA 2337282
(43) Date of publication of application: 21.04.2004
(73) Proprietor: Daykin, Victor Alexander, Pickering, Ontario L1V 3Z5 (CA)
(72) Inventor: Daykin, Victor Alexander, Pickering, Ontario L1V 3Z5 (CA)
(74) Representative: Brooks, Nigel Samuel
(86) International application number: PCT/CA2002/000268
(87) International publication number: WO 2002/070644

(56) References cited:
- EP-A- 0 366 826
- DE-A- 10 011 310
- DE-A- 19 900 683
- GB-A- 2 059 992

## Description

The present invention is directed to a biological specimen collection apparatus which enables the collection of sensitive biological specimens with reduced risk of possible contamination.

### BACKGROUND OF THE INVENTION

The array of diagnostic assays available for the determination of altered or disease states is numerous. The assays include methods for the determination of the presence of a biological marker indicative of the altered or diseased states. These assays generally require the collection of a biological sample from the patient in order to determine the presence of the marker for the altered or diseased state. Many of these assays require that the biological sample be a tissue, cell or other biological specimens. These biological samples are generally collected using a medical swab commonly comprised of a fibrous swab tip on one end of an elongated stick or shaft which is manipulated to contact the swab tip with the source of the biological sample, for example, within the ear, nose or throat of a patient. As a result of swiping the swab against the area of the patient's body, some of the biological sample adheres to the swab tip. The swab tip containing the biological sample is then either contacted with selected reagents which react with the sample to indicate the presence of the marker for the altered or diseased state or placed in a suitable container for transport to a laboratory where further diagnostic tests will be conducted on the sample.

A number of diagnostic assays require that the samples to be assayed be collected in a manner to reduce any possibility of contamination by extraneous material. This is especially true of samples to be assayed by genetic screening methods. In a number of these methods the amount of genetic material in the sample to be assayed may be extremely low and the genetic material must be amplified before the presence of the relevant sequence of genetic material may be determined. In these circumstances, it is important that the sample not be contaminated by extraneous material as the presence of such material may interfere with the assay methodology.

When collecting the sample, some handling of the specimen collection apparatus is required. Such handling risks the introduction of extraneous material into the sample and may affect the results of the assay. In addition, the storage and handling of the sample from the time it is obtained from the subject until the assay is performed may also impact on the test results. Improper handling or storage of the sample may result in the loss of the relevant material in the sample through degradation or inactivation. Some of the factors which may impact sample integrity during storage and transport include environmental factors such as storage temperature and time as well as the presence of potentially harmful energy sources including electromagnetic forces, including the presence of light, particularly UV light. In addition extraneous material or even the sample itself may contain other materials which may act to degrade or inactivate any relevant material in the sample, such as enzymes which would affect the relevant material, for example nucleases for samples containing genetic material etc.

A number of swab collection devices have been proposed in an effort to both improve the contact of the swab with a specific reagent, as well as to maintain the integrity of the sample during post collection transport to a suitable laboratory. For example, US Patent 4,707,450 describes specimen collection test units with a fibrous swab tip at one end and an elongated hollowed shank for use in collecting a biological sample. The shank is carried by a elongated base of a resilient plastic material and contains one or more reagents which can be pumped through the shank to the swab tip by applying manual pressure to the base. An elongated cap removably fits onto the base over the swab shank and tip with a cap including an additional reagent well into which the reagent can be delivered for contact with the swab tip.

US Patent 5,100,028 describes a flexible fluid dispenser having a flexible fluid containing vessel with a seal which seals a top wall of the vessel to a bottom wall and is shaped to concentrate into a region forces resulting in pressure generated by applying a force to the dispenser. In one embodiment, the dispenser is provided with a fibrous swab on the end containing the weaker wall of the vessel which ruptures or opens upon applying the force and saturates the surface with the contents of the vessel.

US Patent 5,078,968 describes a test unit for use in collection and analysis of biological samples. The test unit includes a swab adapted for collection of the specimen to be tested and further include one or more reagents or other test fluids for delivery into contact with the sample on the swab member. The contacted specimen and fluid are delivered through one or more porous filtered members for analysis and/or to contact an additional reagent or reagents in the course of performing a selected test.

US Patent 5,266,266 describes a specimen test unit for use in collection and analysis of a biological sample. The test unit includes a swab having a hollow shaft closed at a rear end by a break off nib and a swab tip at the front end for collecting the selected samples. The swab shaft rear end is carried by a housing base with a break off nib extending into a reagent chamber having a selected reagent therein. The housing base is sufficiently deformable to break the nib and thereby permit reagent delivery through the swab shaft and contact the specimen on the swab tip. Other examples of biological sample collection units are described in US Patents 5,238,649, 5,869,003, 5,879,635.

European Patent No. 0 366 826 NCS Diagnostics Inc, describes a swab applicator, a containment vial and a cap for the vial. The vial contains a culture-preserving medium. The swab applicator has a long shaft with a first section having a length corresponding approximately to the length of the vial and a second section joined to the first section be a frangible joint. After using the applicator, it is inserted into the vial and the second section is broken off. The cap is then treaded into the vial to seal it and capture the first section. In this system, the cap is provided with a central bore, which snugly fits the end of the applicator.

GB 2 059 992 Pike, describes a culture collecting kit having two compartments divided by a rupturable seal. The first compartment contains a growth medium. The second compartment contains a swab having a collecting head. To use this apparatus, a seal is unpeeled, the swab is removed and the culture sample is taken. The package is then torn across the notches and the swab is re-inserted into the rest of the package so that the head penetrates through the seal into the growth medium. The stick portion of the swab is broken off at this point and the package is resealed by an identification lable. As the packaging is of film material, there is a risk of contamination by the end of the swab penetrating the film and allowing external air into the film.

There thus remains a need for a biological specimen collection apparatus that will enable the efficient collection, storage and transport of highly sensitive biological samples without affecting the integrity of the sample by reducing the possibility of extraneous material being introduced into the sample.

### SUMMARY OF THE INVENTION

The present invention provides in one aspect for a biological specimen collection apparatus comprising a biological specimen sampler and a storage and transport container. The biological specimen sampler has a means for holding the sampler by a person taking the sample from a subject and a biological specimen sample collection zone located distal of the holding means, the sample collection zone being separable from the holding means. The storage and transport container is provided in its interior with a means for gripping the sample collection zone to separate the sample collection zone from the holding means such that only the sample collection zone is retained in the interior of the storage and transport container.

In an aspect of the invention there is provided a storage and transport container having in its interior a means for gripping a sample collection zone of a biological specimen sampler to separate the sample collection zone from the biological specimen sampler such that only the sample collection zone is retained in the interior of the storage and transport container.

In another aspect of the invention there is provided a sealing cap for a storage and transport container for a biological sample, the sealing cap having a reagent dispenser integrated therewith, the reagent dispenser being capable of dispensing reagent into the container in a controlled manner.

### BRIEF DESCRIPTION OF THE DRAWINGS

Preferred embodiments of the present invention are illustrated in the attached drawings in which
Figure 1 is a side elevation view of a transport container for use with the apparatus of the present invention;
Figure 2 is a side elevation view in cross section of the transport container of Figure 1;
Figure 3 is a side elevation view of a closure cap with an integrated reagent dispenser for use in a preferred embodiment of the specimen collection apparatus in the present invention;
Figure 4 is a perspective view illustrating the obtaining of a biological sample of a naso-pharyngeal swab utilizing the apparatus of the present invention;
Figure 5 illustrates the insertion of the swab into the storage and transport container;
Figure 6 illustrates the use of the gripping means of the storage and transport container for removing the swab end;
Figure 7 illustrates the attachment of the cap with the integrated reagent dispenser onto the storage and transport container;
Figure 8 illustrates the dispensing of the reagent from the reagent dispenser into the storage and transport container; and
Figure 9 illustrates the combination of the storage and transport container placed within a further tube.

### DETAILED DESCRIPTION OF THE PREFERRED EMBODIMENTS

The biological specimen collection apparatus of the present invention enables the collection of sensitive biological specimens with reduced risk of possible contamination. The apparatus includes a biological specimen sampler and a storage and transport container. In a preferred embodiment, the apparatus further includes a sealing cap for the storage and transport container having a reagent dispenser integrated therewith.

The biological specimen sampler has a means for enabling the sampler to be held by a person taking the sample from a patient and a biological specimen sample collection zone located distal of the holding means. The sample collection zone of the biological specimen sampler is separable from the holding means. The storage and transport container is provided in its interior with a means for gripping the sample collections zone to separate the sample collection zone from the holding means, such that only the sample collection zone is retained in the interior of the storage and transport container. In this way, the potential for contamination of the biological sample by extraneous substances is reduced.

A preferred embodiment of a biological specimen sampler is illustrated in the Figures generally indicated by numeral 10. As illustrated in the Figures the biological specimen sampler 10 is preferably a medical swab having a handle 12 for holding the sampler 10 by a person taking the sample from a patient and a biological sample collection zone, such as the swab tip 14 of a suitable fibrous material for entrapping tissue or cells from the biological sample when the swab tip 14 is contacted with the patient. As further illustrated in the Figures, the handle 12 of the biological specimen sampler 13 preferrably has a bend 16 to enable the swab tip 14 to be more easily placed in contact with the suitable surfaces of the patient, particularly, for naso-pharyngeal, throat or nose swabs. This bending of the handle 12 also aids in the collection of the sample, as a larger area of the surface of the patient may be swept by the swab tip 14 simply by rotating the handle 12 at the end distal of the swab tip 14. Thus, a larger sample area may be contacted with simple and minimal manipulation by the user.

The swab tip 14 is preferably constructed of a suitable fibrous material with sufficient abrading property to remove and entrap cells and tissue from the sample collection zone of the patient. The fibrous material may be any material commonly utilized in the sample swab such as an open cell polymeric foamed or fibrous material, a molded polymeric brush, a cotton or other fabric swab, etc. Preferrably, escpecially for collection of tissue or cells containing genetic material to be analyzed, the fibrous material is an open cell foamed polymeric material. In order to aid in the removal of the swab tip 14 from the handle 12, as will be explained further below, the swab tip 14 is preferably attached to the handle 12 only at the neck 18 of the swab tip 14, rather than along its entire contact area with the handle 12 as is common in the art. This may be accomplished by providing a suitable biocompatible adhesive to adhere just the neck 18 of the swab tip 14 to the handle 12 or the swab tip 14 may be attached to the handle 12 by electrosonic welding in the region of the neck 18 of the swab tip 14. Alternatively, the swab tip 14 may be provided as a molded structure, which is releasably engaged with the end of the handle 12.

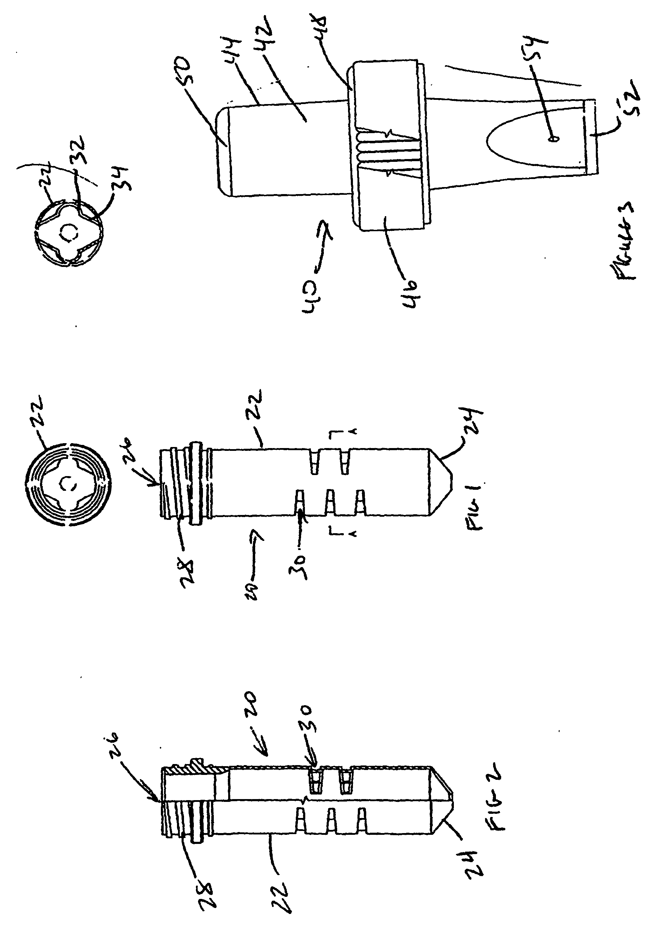

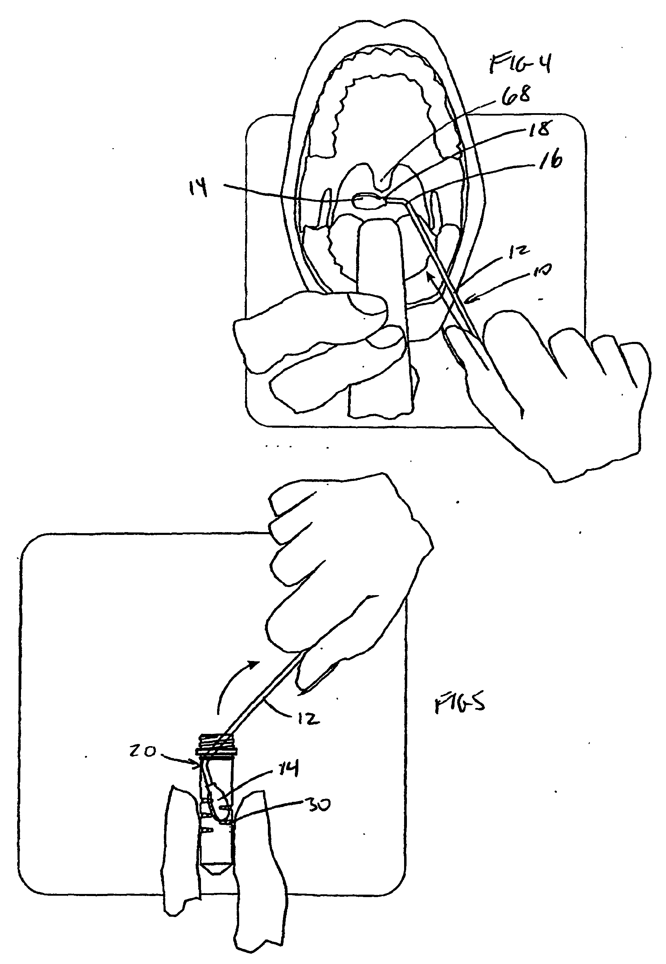

A second component of the biological specimen collection apparatus of the present invention is the storage and transport container 20 provided in its interior with a means for gripping the sample collection zone or swab tip 14 to separate the swab tip 14 from the handle 12 as will be explained further below. A preferred embodiment of a storage and transport container 20 is illustrated in Figures 1 and 2. The storage and transport container 20 is preferably a tube of a suitable size for containing the swab tip 14. For most applications, a tube having an internal capacity of 2 to 5 ml is to be preferred.

As illustrated in the Figures, the storage and transport container 20 has generally straight sides 22 and a conical or rounded bottom 24. The open end 26 of the container 20 is provided with a means for releasably securing a cap 40 thereon for sealing the contents of the container 20. This means for releasably securing the cap 40 may be any such means commonly used in the art, such as a snap fit or threaded connection 28. Preferably, as illustrated in the Figures, the means for securing the cap 40 is a threaded connection 28.

As is also illustrated in the Figures, in the storage and transport container 20 of the preferred embodiment the means for gripping the swab tip 14 is provided by a plurality of inwardly facing projections 30 on opposing sides of the storage container 20. As illustrated in the figures, these inwardly directed projections 30, have a central semi-circular portion 32 with generally straight arms 34 extending from either side of the semi-circular portion 32 and joining adjacent sidewalls of the container 20. This shaping of the inward projections 30 allows the container 20 to be gripped from either the direction aligning with the projections 30 or the direction perpendicular to the projections 30, to grip and remove the sample swab tip 14. As is illustrated in the figures, preferably the projections 30 on the opposite sides of the container 20 are offset to one another such that the projections 30 on one side of the container 20 line up with the space between the projections 30 on the other sides of the sample tube to thereby allow them to interweave.

In order to enable the storage and transport container 20 to be manipulated as necessary to remove the swab tip 14, the container 20 is constructed of a material which allows the container 20 to be deformed without affecting the structural integrity of the container 20 such that the manipulated container 20 will not allow the contents to leak out. Preferrably, the container 20 is molded of a polyethylene polymer having the requiste deformable properties.

The cap 40 for the storage container 30 of the present invention is preferably provided with an integral reagent dispenser 42, which allows reagent to be dispensed into the storage container 20 in a controlled manner once the cap 40 is sealed to the container 20 containing the swab tip 14 as will be explained below. Preferrably, the reagent dispenser 42 is constructed in accordance with the teaching of US Patent No. 5,100,028, the disclosure of which is incorporated herein by reference. The reagent dispenser 42 has a side wall 44 to which is attached or molded the threaded portion 46 of the cap 40 such that the side wall 44 preferrably extends above and below the top surface 48 of the threaded region 46. The top of the reagent dispenser is sealed by a top surface 50 which is secured to the side wall 44 or molded integral therewith. The bottom of the reagent dispenser 42 which is located in the interior of the storage and transport container 20 when the cap 40 is attached thereto, is provided with a sealing weld 52 to retain the reagent within the reagent container 42. The bottom of the reagent container just above the sealing weld 42 is provided with a reduced strength region 54 shaped to concentrate into the region 54 forces resulting in pressure generated by applying a force to the reagent dispenser 42. In the embodiment illustrated, the reduced strength region 54 is a partial circular weld which weakens the wall of the reagent dispenser 42 and which ruptures or opens upon applying the force and allows the contents of the reagent dispenser to be dispensed into the interior of the storage and transport container. It is preferred if the cap and reagent dispenser are also molded of a suitable resilient polymeric material such as polyethylene.

The selection of the reagent contained in the reagent container 42 will depend upon the nature of the assay for which the sample is being collected. For some tests the reagent will be selected to react directly with the sample and give a visual or otherwise easily measureable result. In these situations, the reagent dispenser will contain the reactive reagent. For other tests, it will be necessary to transport the sample to a suitable diagnostic laboratory. In these situations the reagent dispenser will contain a suitable dispensible transport medium or buffer to maintain the sample integrity during transport.

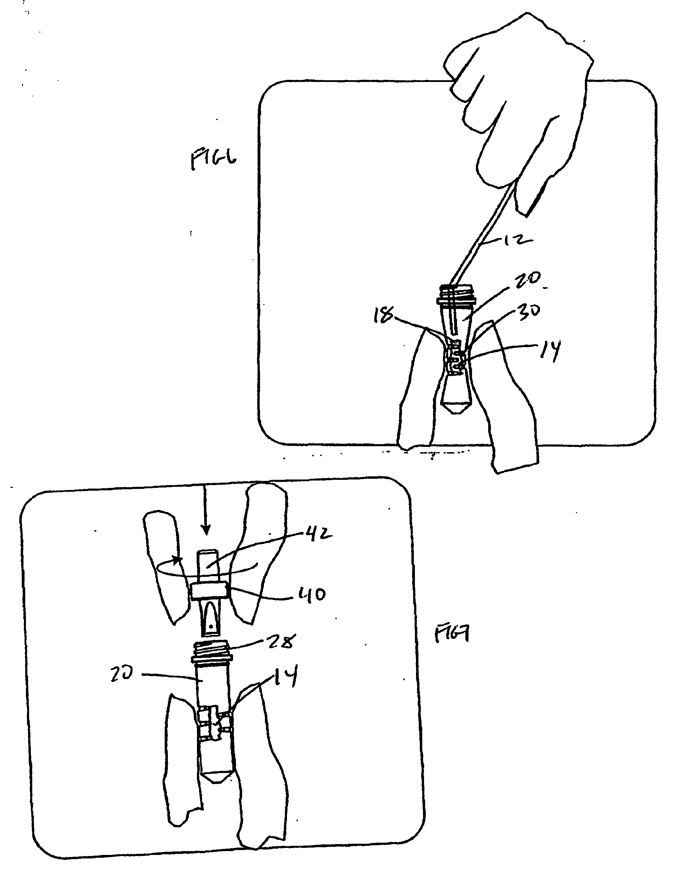

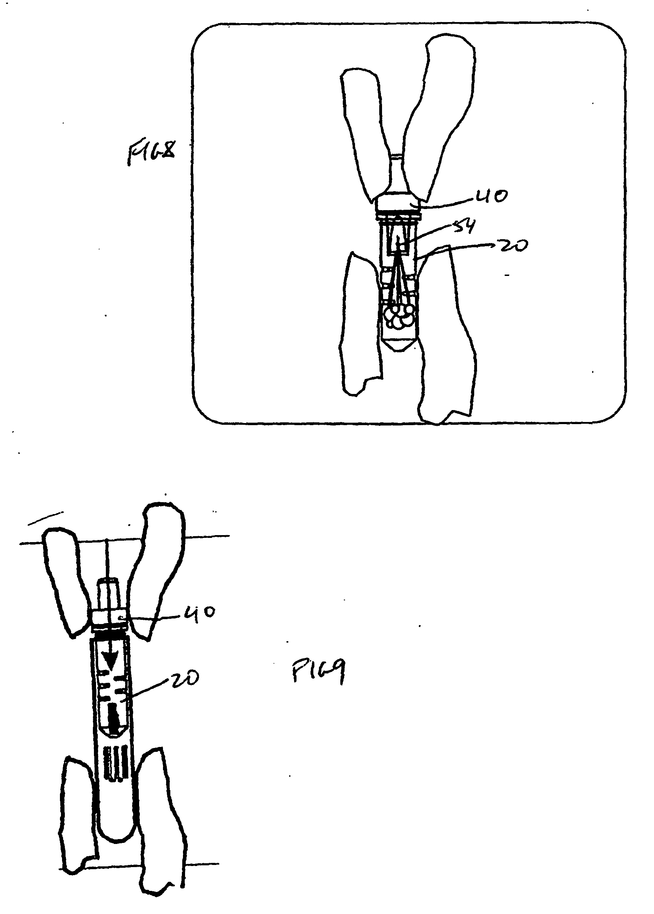

Figures 4 through 8 illustrate the use of the biological specimen collection apparatus of the present invention for the obtaining of a biological sample. In the figures, the apparatus is utilized for obtaining a nasal pharyngeal swab. As shown in figure 4, after removing the biological specimen sampler 10 from a sterile packaging by gripping the handle end 12, a tongue depressor 60 is utilized to gently depress the patient's tongue 62, thereby allowing the palatal arch 64 and oral pharynx 66 to be visualized. The sampler 10 is advanced with the bent tip 14 positioned horizontally into the oral pharyngeal area below the uvula 68. During this procedure, it is preferred to minimize or avoid contact with the oral pharynx 66 to reduce any possible gag reflex. Once the swab tip 14 is in the proper position, the tip 14 is rotated from horizontal to vertical and lifted into the nasopharynx between the uvula 68 and the tonsillar folds. Sampling of the nasopharynx is accomplished with a gentle forward pressure and with two or three firm wiping motions on the bilateral posterior walls of the nasopharynx by simply rotating the handle 12 in the hand. Once the sample is obtained, the tip 14 is lowered from the nasopharynx, rotated back to the horizontal position and withdrawn from the oral pharynx 66.

The storage and transport container 20 is then removed from its sterile packaging and the tip 14 of the sampler 10 placed into the interior of the container 20 until the swab tip 14 aligns with the gripping means provided by the projections 30. The container 20 is then squeezed to allow the projections 30 to tightly engage the tip 14 of the sampler 10. While holding the container 20 in a manner to tightly grip the tip 14, the handle 12 is withdrawn, which results in the tip 14 being stripped from the handle 12. The container 20 is then capped utilizing the cap 40 with the integrated reagent container 42, sealed and the reagent dispensed into the vial. The sealed storage and transport container 20 may then be placed into a larger tube compatible with automatic diagnostic equipment for use in the equipment. This nay be done at the time of collecting the sample and the larger tube sealed for transport to the diagnostic laboratory.

The biological specimen collection apparatus of the present invention enables the efficient collection, storage and transport of highly sensitive biological samples without affecting the integrity of the sample by reducing the possibility of extraneous material being introduced into the sample. Since the apparatus allows the sample collection zone or swab tip of the biological specimen sampler to be removed from the sampler, any possible contamination of extraneous material which may be on surfaces of the sampler are not placed or retained within the storage and transport container. This reduces or eliminates possible contamination of the sample by extraneous material.

## Claims

1. A biological specimen collection apparatus comprising a biological,specimen sampler and a storage and transport container, the biological specimen sampler having means for holding the sampler by a person taking the sample from a subject and a biological specimen sample collection zone located distal of the holding means, the sample collection zone being separable from the holding means, the storage and transport container is provided in its interior with a means for gripping the sample collection zone to separate the sample collection zone from the holding means such that only the sample collection zone is retained in the interior of the storage and transport container.

2. A storage and transport container for a biological sample having in its interior a means for gripping a sample collection zone of a biological specimen sampler to separate the sample collection zone from the biological specimen sampler such that only the sample collection zone is retained in the interior of the storage and transport container.

3. A storage and transport container according to claim 2 further including a sealing cap having a reagent dispenser integrated therewith, the reagent dispenser being capable of dispensing reagent into the container in a controlled manner.

## Patentansprüche

1. Sammeleinrichtung für biologische Proben, mit einem Probennehmer für biologische Proben und mit einem Aufbewahrungs- und Transportbehälter, wobei der Probennehmer für biologische Proben Mittel zum Halten des Probennehmers durch eine Person aufweist, die die Probe von einem Subjekt entnimmt, und eine Probensammelzone aufweist, die sich entfernt von dem Haltemittel befindet, wobei die Probensammelzone von dem Haltemittel abtrennbar ist, wobei der Aufbewahrungs- und Transportbehälter in seinem Inneren mit Mitteln zum Ergreifen der Probesammelzone versehen ist, um die Probensammelzone von dem Haltemittel zu trennen, so dass nur die Probensammelzone im Inneren des Aufbewahrungs- und Transportbehälters zurückgehalten wird.

2. Aufbewahrungs- und Transportbehälter für eine biologische Probe, der in seinem Inneren Mittel zum Erfassen einer Probensammelzone eines Probennehmers für biologische Proben aufweist, um die Probensammelzone von dem Probennehmer für biologische Proben zu separieren, so dass lediglich die Probensammelzone im Inneren des Aufbewahrungs- und Transportbehälters verbleibt.

3. Aufbewahrungs- und Transportbehälter nach Anspruch 2, ferner aufweisend eine Verschlusskappe mit einem integrierten Reagenzspender, wobei der Reagenzspender in der Lage ist, ein Reagenz in den Behälter in kontrollierter Weise abzugeben.

## Revendications

1. Appareil pour recueillir des échantillons biologiques comprenant un dispositif d'échantillonnage d'échantillons biologiques et un récipient de stockage et de transport, l'échantillonneur d'échantillons biologiques ayant un moyen pour retenir l'échantillonneur par une personne prenant l'échantillon d'un sujet et une zone de recueil d'échantillons biologiques située de façon distale au moyen de retenue, la zone de recueil des échantillons étant apte à être séparée du moyen de retenue, le récipient de stockage et de transport est muni dans son intérieur d' un moyen pour saisir la zone de recueil d'échantillons pour séparer la zone de recueil d'échantillons du moyen de retenue afin que seulement la zone de recueil d'échantillons soit retenue dans l'intérieur du récipient de stockage et de transport.

2. Récipient de transport et de stockage pour un échantillon biologique ayant dans son intérieur un moyen pour saisir une zone de recueil d'échantillons d'un échantillonneur d'échantillons biologiques pour séparer la zone de recueil d'échantillons de l'échantillonneur d'échantillons biologiques afin que seulement la zone de recueil d'échantillons soit retenue dans l'intérieur du récipient de stockage et de transport.

3. Récipient de stockage et de transport selon la revendication 2, comprenant, en outre, un capuchon d'étanchéité ayant un dispositif de distribution de réactif intégré dans celui-ci, le distributeur de réactif étant capable de distribuer du réactif dans le récipient d'une façon contrôlée.
